Europäisches Patentamt

European Patent Office

Office européen des brevets

(11) Veröffentlichungsnummer: **0 336 480**
**A1**

(12) **EUROPÄISCHE PATENTANMELDUNG**

(21) Anmeldenummer: 89200759.2

(22) Anmeldetag: 24.03.89

(51) Int. Cl.⁴: **C07C 29/50 , C07C 29/132 , C07C 31/12**

(30) Priorität: 08.04.88 DE 3811849

(43) Veröffentlichungstag der Anmeldung:
11.10.89 Patentblatt 89/41

(84) Benannte Vertragsstaaten:
**DE FR GB IT NL**

(71) Anmelder: **METALLGESELLSCHAFT
AKTIENGESELLSCHAFT
Reuterweg 14 Postfach 10 15 01
D-6000 Frankfurt 1(DE)**

(72) Erfinder: **Witzgall, Konrad, Dr.
Gerhard-Hauptmann-Strasse 11
D-8520 Erlangen(DE)**
Erfinder: **Simo, Thomas, Dr.
Kälberstücksweg 44
D-6380 Bad Homburg(DE)**
Erfinder: **Hofmann, Hanns, Dr., Prof.
Universität Erlangen Egerlandstrasse 3
D-8520 Erlangen(DE)**

(54) **Verfahren zur Herstellung von tertiärem Butanol.**

(57) Tertiäres Butanol (TBA) wird durch katalytische Oxidation von Isobutan mit molekularem Sauerstoff in flüssiger Phase erzeugt. Dabei entsteht ein Rohprodukt, das TBA und u.a. tertiäres Butylhydroperoxid (TBHP) enthält. Aus dem Rohprodukt wird durch destillative Zerlegung TBA gewonnen. Für die Umsetzung verwendet man einen Katalysator, bei dem es sich um eine Übergangsmetallverbindung mit stickstoffhaltigen Liganden oder aber um ein polymeres Phthalocyanin handelt. Der Katalysator ist im Reaktionsgemisch praktisch unlöslich. Vorzugsweise wird das Rohprodukt bei Temperaturen von 100 bis 135° C und bei einem Druck im Bereich von 5 bis 50 bar hergestellt.

EP 0 336 480 A1

## Verfahren zur Herstellung von tertiärem Butanol

Die Erfindung betrifft ein Verfahren zur Herstellung von tertiärem Butanol (TBA) durch Oxidation von Isobutan mit molekularem Sauerstoff in Anwesenheit eines heterogenen Katalysators in flüssiger Phase, wobei ein TBA und tertiäres Butylhydroperoxid (TBHP) enthaltendes Rohprodukt entsteht. Nach Zersetzung des TBHP wird daraus durch destillative Zerlegung TBA gewonnen.

Aus Isobutan $(CH_3)_3CH$, ensteht durch Oxidation tertiäres Butylhydroperoxid, $(CH_3)_3COOH$, aus welchem sich durch Zersetzung des Hydroperoxids TBA bildet, dessen chemische Summenformel $(CH_3)_3COH$ ist. Der Erfindung liegt die Aufgabe zugrunde, dieses Verfahren mit guter Selektivität auszuführen und insbesondere einen guten Katalysator hierfür zu finden. Dies gelingt erfindungsgemäß dadurch, daß der Katalysator im Reaktionsgemisch praktisch unlöslich und

a) eine Übergangsmetallverbindung mit stickstoffhaltigen Liganden

oder

b) ein polymeres Übergangsmetall-Phthalocyanin

ist.

Der beim Verfahren der Erfindung zu verwendende Katalysator besitzt Redoxfähigkeit und soll in allen Oxidationszuständen im Reaktionsgemisch unlöslich bleiben.

Die unter a) genannten Katalysatoren können z.B. Verbindungen der Zusammensetzung M - $(NR_3)$ x · $Yz$ sein, wobei M ein Übergangsmetallion - vorzugsweise Kobalt, Mangan oder Eisen - ist, und R entweder Wasserstoff oder Alkylrest sein kann, und Y ein Säurerest ist und sowohl anorganischer als auch organischer Natur sein kann. Ein Beispiel aus dieser Gruppe sind die Heteropolysäuren und deren Salze. Einzelheiten zur Struktur und Herstellung der Heteropolysäuren und deren Salze sind in Remy, Lehrbuch der anorganischen Chemie (1954), Band 2, Akademische Verlagsgesellschaft, Leipzig, S. 181-187, sowie in der DE-OS 31 14 350 und der Chemiker-Zeitung (1983), S. 211-218, beschrieben.

Bei den unter b) genannten Katalysatoren ist das Übergangsmetall in einer Porphyrinstruktur eingebunden, vorteilhaft als polymeres Phthalocyanin. Zur Struktur und Herstellung von Phthalocyanin und dessen Polymeren wird auf das US-Patent 4 028 423 verwiesen.

Vorteilhafterweise enthält der unter a) oder b) genannte Katalysator mindestens ein Metall der Gruppe VIII (CAS-Version der IUPAC-Tabelle ab 1962) oder VIIB des Periodensystems wie Fe, Ni, Mn oder vorteilhafterweise Co. Außerdem kann Cu aus der Gruppe IB des Periodensystems enthalten sein.

Überraschenderweise wurde gefunden, daß die Konzentration und vor allem die aktive Oberfläche der beschriebenen Katalysatoren den Reaktionsverlauf entscheidend beeinflussen.

Im Gegensatz zu den bekannten Isobutanoxidationsverfahren hat die Verwendung dieser Katalysatoren in geeigneter Konzentration zur Folge, daß die Selektivität zu TBHP vergleichsweise gering ist zugunsten einer hohen Selektivität zu TBA. Dies geschieht durch katalytische Zersetzung des TBHP zu Radikalen, die die TBA-Bildung und gleichzeitig die Nachbildung des TBHP ermöglichen.

Die Herstellung des flüssigen Rohproduktes erfolgt bevorzugt bei Temperaturen im Bereich von etwa 100 bis 135° C. Zweckmäßigerweise wird das Rohprodukt bei einem Druck im Bereich von 5 bis 50 bar hergestellt.

Zur Herstellung des Rohprodukts wird flüssiges Isobutan mit dispergiertem Katalysator und molekularem Sauerstoff bevorzugt fein verteilt in Kontakt gebracht. Der Sauerstoff wird als technisch reiner Sauerstoff oder auch als sauerstoffhaltiges Gasgemisch, z.B. Luft, für die Umsetzung verwendet. Die Reaktion wird zweckmäßigerweise durch Zugabe von TBHP, die hier als Initiatorsubstanz dient, gestartet. Die Umsetzung kann kontinuierlich oder chargenweise erfolgen.

Die Vorteile des Verfahrens kommen besonders bei kontinuierlicher Fahrweise zur Geltung, wobei fortlaufend Isobutan in den Reaktor eingespeist und Reaktionsprodukt abgezogen wird. Im Reaktor wird für intensive Durchmischung gesorgt. Die Verweilzeit wird so bemessen, daß ein Umsatz zwischen 40 und 60 % erreicht wird.

Das Rohrprodukt enthält insbesondere nicht umgesetztes Isobutan sowie das gewünschte TBA und weitere Oxidations-und Zwischenprodukte, vor allem TBHP. Weitere Zwischen-und Nebenprodukte in kleinen Mengen sind beispielsweise Aceton, Methanol, Ameisensäure, Formaldehyd, Wasser und Di-tert.-Butylperoxid (DTBP).

Das Reaktionsprodukt wird z.B. durch Filtration vom Katalysator befreit und durch Zersetzung (z.B. thermisch) werden Peroxide entfernt, wobei sich aus TBHP das gewünschte Endprodukt TBA bildet. Das nicht umgesetzte Isobutan wird abgetrennt und in den Prozeß zurückgeführt.

Ein Teil wird ausgeschleust, um eine unerwünschte n-Butananreicherung im Reaktor zu vermeiden. In weiteren destillativen Trennoperationen wird das Hauptprodukt TBA von leichtsiedenden und schwersiedenden Nebenprodukten getrennt.

Beispiel 1

Ein einer Laborapparatur wird in einem Edelstahlautoklaven mit einem Fassungsvermögen von 500 ml, der elektrisch heizbar ist, das Rohprodukt in allen Fällen bei 130° C und 50 bar Druck hergestellt. Es werden 250 ml Isobutan eingesetzt; die Menge an körnigem Katalysator beträgt 300 g, als Initiatorsubstanzen werden 8 ml TBHP und 2 ml Di-tert.-Butylperoxid verwendet. Unter ständigem Rühren mit einem Magnetrührer leitet man Luft durch den Autoklaven.

Es werden vier verschiedene Katalysatoren - A, B, C und D - verwendet, wobei die Katalysatoren A und B die erfindungsgemäße Zusammensetzung haben, und die beiden anderen Katalysatoren nur Vergleichszwecken dienen.

Katalysator A ist Di-Hexamin-Kobalt(II)-Di-vanadato-10-molybdatophosphorsäure mit der chemischen Summenformel

$H[Co(NH_3)_6]_2Mo_{10}V_2PO_{40} \cdot xH_2O$.

Katalysator B ist Kobalt(II)-phthalocyanin-polymer.

Katalysator C ist Molybdänacetylacetonat.

Katalysator D ist Kobalt(II)-carbonat basisch mit der chemischen Summenformel $CoCO_3[Co(OH)_2]_n \cdot xH_2O$.

Die Zusammensetzung des Rohproduktes nach einer Umsetzungszeit von 1 h, 3 h und 7 h bei Verwendung der vier verschiedenen Katalysatoren ist in der nachfolgenden Tabelle dargestellt. Dabei bedeutet $\Delta iC4$ den Anteil, in Gew.-%, an umgesetztem Isobutan, TBA bedeutet den Anteil im Rohprodukt an TBA, in Mol.-%, bezogen auf das umgesetzte Isobutan, und AC bedeutet den Anteil im Rohprodukt an Aceton, in Mol.-%, ebenfalls bezogen auf das umgesetzte Isobutan. Der Anteil an Aceton ist charakteristisch für die Nebenproduktbildung.

|  | t = 1 Stunde | | | t = 3 Stunden | | | t = 7 Stunden | | |
|---|---|---|---|---|---|---|---|---|---|
|  | $\Delta iC4$ | TBA | AC | $\Delta iC4$ | TBA | AC | $\Delta iC4$ | TBA | AC |
| Kat.A | 28,7 | 43,9 | 2,6 | 41,3 | 52,2 | 5,0 | 58,0 | 65,9 | 9,0 |
| Kat.B | 14,7 | 44,7 | 4,9 | 28,22 | 63,5 | 5,9 | 60,5 | 54,7 | 7,0 |
| Kat.C | 26,0 | 6,0 | 7,1 | 27,2 | 3,9 | 9,5 | - | - | - |
| Kat.D | 11,4 | 36,5 | 6,3 | 15,3 | 54,3 | 11,0 | 22,2 | 63,6 | 11,4 |

Beim Versuch mit Katalysator A sind der hohe Umsatz und die relativ hohe Selektivität der Bildung von TBA bemerkenswert. Das Reaktionsgemisch ist selbst nach langen Reaktionszeiten vollkommen klar und der Katalysator unverändert.

Katalysator B wird als pulverförmige Substanz ohne vorheriges Pressen eingesetzt, der Katalysator ist nach der Reaktion unverändert.

Die Aktivität des Katalysators C geht schnell verloren, so daß die Untersuchungen nach 3 h Umsetzungszeit abgebrochen wurden.

Der Katalysator D erbrachte nur einen geringen Umsatz.

Beispiel 2

In einer ähnlichen Apparatur wie im Beispiel 1 wurde ein kontinuierlicher Versuch mit polymerem Co-Phthalocyanin (Katalysator B) bei einer Temperatur von 130° C und einem Druck von 50 bar durchgeführt. Der Rohstoff floß dem Reaktor kontinuierlich von oben zu, das Reaktionsprodukt wurde kontinuierlich über ein Tauchrohr abgezogen. Weiter herrschten folgende Bedingungen:

| Katalysatorkonzentration: | 1,36 | g/l |
|---|---|---|
| Flüssigkeitsvolumen im Reaktor: | 300 | ml |
| zugeführte Isobutan-Menge: | 30 | g/h |
| zugeführte $O_2$-Menge: | 5,2 | g/h |
| zugeführte $N_2$-Menge: | 3 | g/h |

Das abgezogene Reaktionsprodukt wurde laufend analysiert.

Die nachfolgende Tabelle, aufgenommen nach einer Versuchsdauer von 20 h bis zu 30 h, zeigt die Umsetzung des Isobutans zu den verschiedenen Produkten. Kleine Abweichungen in der Bilanz sind auf unvermeidbare Meßungenauigkeiten zurückzuführen.

| | Einsatz | Flüssigphase Ausgang | Gasphase Ausgang |
|---|---|---|---|
| Isobutan | $8,400 \bullet 10^{-3}$ mol/min | $4,182 \bullet 10^{-3}$ mol/min | $2,751 \bullet 10^{-4}$ mol/min |
| | 0,488 g/min | 0,243 g/min | 0,016 g/min |
| TBA | ------ | $3,039 \bullet 10^{-3}$ mol/min | ------ |
| | | 0,225 g/min | |
| Aceton | ------ | $3,087 \bullet 10^{-4}$ mol/min | ------ |
| | | 0,0179 g/min | |
| Methanol | ------ | $1,642 \bullet 10^{-4}$ mol/min | ------ |
| | | 0,005 g/min | |
| TBHP | ------ | $4,387 \bullet 10^{-4}$ mol/min | ------ |
| | | 0,040 g/min | |
| DTBP | ------ | $5,687 \bullet 10^{-5}$ mol/min | ------ |
| | | 0,008 g/min | |
| $O_2$ | $2,681 \bullet 10^{-3}$ mol/min | ------ | $2,361 \bullet 10^{-4}$ mol/min |
| | 0,086 g/min | | 0,008 g/min |
| $N_2$ | $1,791 \bullet 10^{-3}$ mol/min | ------ | $1,791 \bullet 10^{-3}$ mol/min |
| | 0,050 g/min | | 0,050 g/min |
| $CO_2$ | ------ | ------ | $5,7 \bullet 10^{-5}$ mol/min |
| | | | 0,0025 g/min |
| $H_2O$ | ------ | ------ | $2,5 \bullet 10^{-5}$ mol/min |
| | | | $4,5 \bullet 10^{-4}$ g/min |

## Ansprüche

1. Verfahren zur Herstellung von tertiärem Butanol (TBA) durch Oxidation von Isobutan in Gegenwart eines Katalysators mit molekularem Sauerstoff in flüssiger Phase, wobei ein TBA und tertiäres Butylhydroperoxid (TBHP) enthaltendes Rohprodukt entsteht, aus dem nach Zersetzung des TBHP durch destillative Zerlegung TBA gewonnen wird, dadurch gekennzeichnet, daß der Katalysator im Reaktionsgemisch praktisch unlöslich und

a) eine Übergangsmetallverbindung mit stickstoffhaltigen Liganden

oder

b) ein polymeres Übergangsmetall-Phthalocyanin

ist.

2. Verfahren nach Anspruch 1, dadurch gekennzeichnet, daß der Katalysator mindestens ein Metall der Gruppe VIIB oder der Gruppe VIII des Periodensystems enthält.

3. Verfahren nach Anspruch 1, dadurch gekennzeichnet, daß der Katalysator mindestens ein Metall der Gruppe IB (Cu, Ag, Au) des Periodensystems enthält.

4. Verfahren nach Anspruch 1 oder einem der folgenden, dadurch gekennzeichnet, daß der unter a) genannte Katalysator als Anion eine Heteropolysäure mit Phosphor als Zentralatom enthält.

5. Verfahren nach Anspruch 4, dadurch gekennzeichnet, daß die Heteropolysäure mindestens ein Element der Gruppe VB oder VIB des Periodensystems koordiniert enthält.

6. Verfahren nach Anspruch 1, dadurch gekennzeichnet, daß das polymere Phthalocyanin Kobalt als Zentralatom enthält.

7. Verfahren nach Anspruch 1 oder einem der folgenden, dadurch gekennzeichnet, daß der Katalysator zusammen mit einer Trägersubstanz angewendet wird.

8. Verfahren nach Anspruch 1 oder einem der folgenden, dadurch gekennzeichnet, daß das Rohprodukt bei Temperaturen von 100 bis 135°C hergestellt wird.

9. Verfahren nach Anspruch 1 oder einem der folgenden, dadurch gekennzeichnet, daß das Rohprodukt bei einem Druck im Bereich von 5 bis 50 bar hergestellt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 816 548 (R.H. WILLIAMS) <br> * Spalten 1-6 * <br> --- | 1-3,6-9 | C 07 C 29/50 <br> C 07 C 29/132 <br> C 07 C 31/12 |
| A | GB-A-1 166 121 (EDISON INTERNATIONAL CORP.) <br> * Ansprüche 1-6; Seite 2, Zeilen 25-38; Seite 3, Zeilen 31-36 * <br> --- | 1,4,5 | |
| A | GB-A-1 212 824 (IMPERIAL CHEMICAL INDUSTRIES LTD) <br> * Ansprüche; Seite 1, Zeilen 24-34 * <br> --- | 1 | |
| A | GB-A-1 428 964 (IMPERIAL CHEMICAL INDUSTRIES LTD) <br> * Ansprüche * <br> --- | 1 | |
| P,A | EP-A-0 308 101 (TEXACO DEVELOPMENT CORP.) <br> * Seiten 4,5; Ansprüche * <br> ----- | 1 | |

**RECHERCHIERTE SACHGEBIETE (Int. Cl.4)**

C 07 C 29/00

Der vorliegende Recherchenbericht wurde für alle Patentansprüche erstellt

| Recherchenort | Abschlußdatum der Recherche | Prüfer |
|---|---|---|
| DEN HAAG | 21-06-1989 | BONNEVALLE E.I.H. |